# EUROPEAN PATENT APPLICATION

(11) **EP 3 076 168 A1**
(43) Date of publication of application: **05.10.2016**
(21) Application number: 15162096.0
(22) Date of filing: 31.03.2015
(51) Int. Cl.: G01N 27/404, G01N 33/00

(54) **An electrochemical sensor**

(71) Applicant: Unisense Environment A/S, 8200 Aarhus N (DK); Unisense A/S, 8200 Aarhus N (DK)
(72) Inventor: Sveegaard, Steffen Gralert, 9230 Svenstrup J (DK); Nielsen, Michael, 8260 Viby J (DK)
(74) Representative: Plougmann Vingtoft a/s

(57) **Abstract**

There is presented an electrochemical sensor (100) for sensing an analyte in an associated volume (106), the sensor comprising a sensing chamber (120), the sensing chamber (120) comprising, a working electrode (104), wherein the electrochemical sensor (100) comprises an aprotic solvent comprising an oxygen scavenger, said aprotic solvent comprising said oxygen scavenger being arranged for hindering oxygen in passing from the associated volume to the working electrode (104). In a particular embodiment the oxygen scavenger is a phosphine.

## Description

### FIELD OF THE INVENTION

The present invention relates to electrochemical sensors and more particularly to electrochemical sensors for sensing analytes, such as nitrous oxide, and corresponding methods and use.

### BACKGROUND OF THE INVENTION

For numerous purposes, such as environmental monitoring, biological research, or wastewater treatment, it is beneficial to be able to sense or quantitatively measure analytes, such as nitrous oxide. Sensors, such as sensors for measuring nitrous oxide, have previously been proposed.

A electrochemical sensor has been proposed for example in the patent application WO2014101921A1.

An improved sensor would be advantageous, and in particular a more efficient, sensitive, durable, compact and/or reliable sensor would be advantageous.

### SUMMARY OF THE INVENTION

It may be seen as advantageous to provide a more efficient, sensitive, durable, compact, stable, temperature insensitive and/or reliable sensor for sensing nitrous oxide. It may also be seen as a further object of the present invention to provide an alternative to the prior art. To better address one or more of these concerns, in a first aspect of the invention there is provided an electrochemical sensor (100) for sensing an analyte in an associated volume (106), the sensor comprising
- a sensing chamber (120), the sensing chamber (120) comprising,
   - A working electrode (104),
   - A reference electrode (108), and
   - An electrolyte, where the sensing chamber (120) has a sensing chamber opening (122), where a sensing chamber opening membrane (124) is placed in the sensing chamber opening (122),
   wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
   wherein
   the electrochemical sensor (100) comprises an aprotic solvent comprising an oxygen scavenger, said aprotic solvent comprising said oxygen scavenger being arranged for hindering oxygen in passing from the associated volume to the working electrode (104).

In an alternative version of the first aspect of the invention there is provided an electrochemical sensor (100) for sensing an analyte in an associated volume (106), the sensor comprising
- a sensing chamber (120), the sensing chamber (120) comprising,
   - A working electrode (104),
   - A reference electrode (108), and
   - An electrolyte,
   where the sensing chamber (120) has a sensing chamber opening (122), where a sensing chamber opening membrane (124) is placed in the sensing chamber opening (122),
wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
wherein
the electrochemical sensor (100) comprises an aprotic solvent comprising an oxygen scavenger.

It may generally be understood, that said aprotic solvent comprising said oxygen scavenger will typically be arranged for hindering oxygen in passing from the associated volume to the working electrode (104), but it may in general also be conceivable and encompassed by the present invention in accordance with alternative versions, that embodiments have said aprotic solvent comprising said oxygen scavenger not necessarily being arranged for hindering oxygen in passing from the associated volume to the working electrode (104).

It may in general also be conceivable and encompassed by the present invention and/or alternative versions that embodiments have said aprotic solvent comprising said oxygen scavenger being arranged for *partially* hindering oxygen in passing from the associated volume to the working electrode (104).

Throughout this application, "pre-chamber membrane" is and/or may be used interchangeably with "pre-chamber opening membrane".

The invention is particularly, but not exclusively, advantageous for obtaining an improved sensor for sensing an analyte, and in particular a more efficient, sensitive, durable, compact and/or reliable sensor. It may in particular be seen as advantageous that when the aprotic solvent comprising the oxygen scavenger enables dispensing with aqueous solutions. A possible advantage of this may be that the possible absence of water in or adjacent the sensing chamber enables reducing or reduces the amount of water at the working electrode, which in turn realizes a relatively low zero-current, which in turn enables improving sensitivity. It may be seen as an insight made by the inventors, that use of an oxygen scavenger in an aprotic solvent enables a high signal-to-noise ratio. The present inventors have also made the insight, that another possible advantage is that a sensor lifetime may be improved, since a transport of water into the sensing chamber may be reduced and since water in the sensing chamber may reduce sensor lifetime. Another advantage may be, that without water, e.g., in a pre-chamber, the sensing chamber opening can be made larger - without increasing a transport of water to a critical level for performance - which in turn enables increased performance (sensitivity and/or response time) and enables more freedom in design (such as dimensions) which in turn enables more freedom in selection of materials for construction of, e.g., the sensing chamber. Another possible advantage of having an aprotic solvent with an oxygen scavenger, may be that it may be comprised within the sensing chamber, i.e., possibly rendering the pre-chamber superfluous, which may in turn be beneficial for one or more of sensitivity, response time and complexity of the sensor.

Furthermore, it should be recognized, that the present inventors have realized through inventiveness and massive research that despite having the electrolyte being based on an aprotic solvent, in the particular sensor described in the independent claim, water vapour may nevertheless find its way to the working electrode and cause a zero-current, such as an elevated zero-current, due to proton reduction. However, having, e.g., a pre-chamber with an aprotic solvent comprising an oxygen scavenger, may reduce or eliminate this probem.

Prior to discussing the present invention in further details, the following terms and conventions will first be defined:
By 'electrochemical sensor' is understood a sensor that detects the presence, such as measure the concentration, of an analyte, such as nitrous oxide (N₂O) or carbon dioxide (CO₂), by oxidizing or reducing the analyte at an electrode and detecting, such as measuring, the resulting current. It is understood that the resulting current need not necessarily be measured as a current, but may for example be measured as a voltage drop across a resistor. The word 'sensor' and 'electrochemical sensor' are generally used interchangeably within the context of the present application. An example of an electrochemical sensor is described in the article "An oxygen insensitive microsensor for nitrous oxide", by Knud Andersen, Thomas Kjaer and Niels Peter Revsbech, Sensors and Actuators B 81 (2001) 42-48, which is hereby incorporated by reference in entirety, and a particular reference is made to section 2.1 describing sensor construction. Another example of an electrochemical sensor is described in the patent application WO2014101921A1, which is hereby incorporated by reference in entirety.

By 'analyte' is understood the compound of interest, such as a molecule, such as nitrous oxide or carbon dioxide.

By 'sensing' is understood qualitatively detecting the presence of an analyte. In some more specific embodiments, sensing may be construed as quantitatively measuring a concentration of an analyte.

By 'associated volume' is understood an associated volume which is adjacent the sensor and which may contain the analyte, such as nitrous oxide. The associated volume is not to be construed as limiting to the scope of the claims. The concentration of the analyte in the associated volume may be measured with the sensor. The associated volume may comprise a fluid, a gas or a matrix, such as a biofilm, such as an extracellular matrix.

By 'sensing chamber' is understood a chamber, such as a casing, which delimits a sensing chamber volume within the sensing chamber from the surroundings external to the sensing chamber. However, it is encompassed by the present invention, that the sensing chamber may have one or more through-going holes in the delimiting walls, such as openings for electrical wiring or membranes. However, in general, the sensing chamber does not allow fluid passage from outside the sensing chamber to inside the sensing chamber. In particular embodiments, the sensing chamber may comprise walls made at least partially of glass. In particular embodiments, the sensing chamber may comprise walls made at least partially of a polymeric material, such as polyether ether ketone (PEEK).

By 'sensing chamber opening' is understood a through-going hole in the walls of the sensing chamber which through-going opening connects the external volume (which may in embodiments comprise the pre-chamber volume and in other embodiments, e.g., embodiments without a pre-chamber, may correspond to the associated volume) with a sensing chamber volume within the sensing chamber.

By 'sensing chamber membrane' is understood a membrane material which is placed in the sensing chamber opening. The sensing chamber membrane is arranged so as to separate the external volume (which may in embodiments comprise the pre-chamber volume and in other embodiments, e.g., embodiments without a pre-chamber, may correspond to the associated volume) from the sensing chamber volume within the sensing chamber. More specifically, the sensing chamber membrane is situated so as to fill the sensing chamber opening, so as to block passage from the external volume (which may in embodiments comprise the pre-chamber volume and in other embodiments, e.g., embodiments without a pre-chamber, may correspond to the associated volume) to the sensing chamber volume of any substance incapable of penetrating through the sensing chamber membrane. It is understood, that the sensing chamber membrane may refer to a thin, film-like structure that separates two fluids, such as a liquid or gas in the external volume (which may in embodiments comprise the pre-chamber volume and in other embodiments, e.g., embodiments without a pre-chamber, may correspond to the associated volume), and a liquid or gas in the sensing chamber volume. However, it is also understood that the sensing chamber membrane may act as a selective barrier, allowing some particles or chemicals to pass through but not others. It may in particular be understood, that the sensing chamber membrane is permeable to nitrous oxide and/or carbon dioxide. The sensing chamber membrane may in particular embodiment comprise, such as consist of, silicone, such as any one of the silicone sealants obtainable from Dow Corning with product number 732 or 734. A possible advantage of using silicone as sensing chamber membrane material may be that it combines good adhesion to glass with high permeability to, e.g., N₂O.

'Working electrode' is known in the art, and understood to be the electrode the electrochemical sensor on which the reaction of interest is occurring. It may be understood that the reduction of nitrous oxide is taking place at the working electrode. It may be understood that the working electrode may be understood as the cathodic electrode or the cathode.

'Reference electrode' is known in the art, and understood to be the electrode the electrochemical sensor on which acts as reference in measuring and controlling the working electrodes potential. It may be understood that the reference electrode may be referred to in the present applications as the anodic electrode or the anode. In particular embodiments there may be used silver (Ag) in or on the reference electrode.

By 'guard electrode' is understood an additional electrode, such as an additional cathode, which is arranged so as to remove oxygen diffusing toward the working electrode from the sensing chamber. In particular embodiments, the working electrode is placed somewhat towards one end of the sensing chamber, such as the end with the sensing chamber opening, such as near the sensing chamber opening, and the guard electrode is placed between the working electrode and a middle portion of the sensing chamber. A guard electrode is described in "An oxygen microsensor with a guard cathode", NP Revsbech, Limnol. Oceanogr., 34(2), 1989, 474-478, which is hereby incorporated by reference in entirety.

By 'electrolyte' is understood a liquid comprising ions. 'Electrolyte' may be used interchangeably with 'electrolyte solution'. The electrolyte is understood to be electrically conducting. It is understood that the electrolyte electrically connects the reference electrode and the working electrode. The charge carriers are dissolved ionic compounds. The electrolyte advantageously comprises an aprotic solvent, which may or may not be identical to the aprotic solvent comprising the oxygen scavenger.

In the context of the present invention "oxygen scavenger" is defined in the broadest sense as any compound capable of removing oxygen molecules from a medium such as a solvent or porous matrix. Advantageous oxygen scavengers are soluble and/or dispersible in a given preferred medium. Oxygen scavengers are often reducing agents or agents who form chemical bonds with oxygen atoms by other means.

In the present context an "organophosphorous compound" is any organic compound comprising a phosphor atom, such as a phosphorous compound derivatised by organic derivatives or hydrogen. In the context of the present invention "phosphine" is defined in the broadest sense as trivalent organophosphorus compounds with the formula R¹-P-(R²)R³ (R = organic derivative or hydrogen). "Phosphine" is however not to be understood as including phosphane gas (PH₃) or other highly volatile phosphines. The organic derivatives may include ionic groups such as ammonium ion and sulfonium ions, and salts thereof. Organophosphorous compounds and phosphines of the present invention must also fulfil the criteria for oxygen scavengers.

In the context of the present invention "alkyl" is defined in the broadest sense as saturated or unsaturated, branched or linear non-aromatic hydrocarbons. They may be optionally substituted by non-hydrocarbons. Heteroalkyls are alkyl with heteroatoms as part of/inserted in the alkyl chain, e.g. CH₃-(CH₂)ₙ-O-(CH₂)ₘ-, where m and n are integers. Herein "cykloalkyls" are cyclic alkyls, which may also include heterocycloalkyls, where a heteroatom is included in the ring. Unsaturated alkyls includes alkenyl comprising C-C double bonds, and alkynyl comprising C-C triple bonds.

In the context of the present invention "aryl" is defined in the broadest sense as aromatic hydrocarbons. Examples include phenyl and napthyl. "heteroaryl" is defined as aromatic groups comprising at least one heteroatom, such as e.g. O, S, or N. Examples of heteroaryls include e.g. pyridine, pyrrole, and thiazole.

In the context of the present invention "non-aqueous solvent" is defined in the broadest sense as any solvent which does not comprise water or comprises extremely low amounts of water, such as below 1000 ppm, below 100 ppm, 50 ppm, 10 ppm, 5 ppm, 1 ppm, such as preferably below 0.1 ppm. It is noted that in the present context of electrochemical sensors non-aqueous solvent may in certain embodiments include porous matrices such as polymers selected from the group consisting of silicone, PVC, polystyrene or latex, and also inorganic porous matrices selected from the group consisting of siliciumdioxide, zirconiumdioxide, titaniumdioxide, alumina, indiumnitride, or boronnitride.

In the context of the present invention "counter-ion" is defined in the broadest sense as a positive or negative ion which forms a positive or negative counter-ion in a salt further comprising an ionic compound of opposite charge. A counter-ion may be inorganic or organic. In the present context the counter-ion will be bound to an ionic oxygen scavenger, such as e.g. a phosphine with an ionic substituent.

In the context of the present invention "aprotic solvent" is defined in the broadest sense as any solvent that does not comprise a labile proton (H⁺), i.e. a solvent that does not readily donate a proton, such as which cannot donate protons. It may preferably be an organic aprotic solvent. Examples of labile protons are protons bonded to oxygen in hydroxy groups or bonded to nitrogen in amine groups. Examples of non-labile protons are e.g. alkyl protons such as -CH₂- group protons. Examples include propylene carbonate, dimethyl sulfoxide (DMSO), acetonitrile (MeCN), dimethylformamide (DMF), acetone, ethyl acetate (EtOAc), tetrahydrofuran (THF). The present inventors have made the insight that an advantage of employing an aprotic solvent in the electrolyte (in the sensing chamber) in the sensor may be that the zero-current is lowered, since less protons is available for reduction into H₂ molecules. In embodiments with a pre-chamber, the present inventors have made the insight that an advantage of employing an aprotic solvent in the pre-chamber, such as a aprotic solvent with an oxygen scavenger (which aprotic solvent with an oxygen scavenger may or may not be similar to the aprotic solvent comprised within the electrolyte) may be that less water enters the sensing chamber, which enables that the zero-current is lowered, since less protons is available for reduction into H₂ molecules.

By 'ionic compound' is understood compounds held together by ionic forces. Ionic compounds may, however, be dissolved in some fluids, such as TBA-I being dissolved in propylene carbonate so as to form TBA ions and iodide ions.

By 'means for hindering oxygen in passing' is understood means which hinders oxygen in passing from the associated volume, such as the outside of the sensor, to the working electrode. The means for hindering oxygen in passing may function by, for example blocking or degrading oxygen before it reaches the working electrode, optionally even before it reaches the sensing chamber volume.

In a specific embodiment, the 'means for hindering oxygen in passing into the sensing chamber volume' comprises 'means for hindering a diffusion of oxygen' by which is understood means which hinders diffusion of oxygen, such as freely diffusing oxygen, from the associated volume, such as the outside of the sensor, to the working electrode. The means for hindering a diffusion of oxygen comprises an aprotic solvent with an oxygen scavenger.

However, the means for hindering oxygen in passing into the sensing chamber volume is not limited exclusively to blocking diffusion of oxygen, but may also in certain embodiments be arranged so as to block passage of oxygen in a sample which is actively led from the associated volume to the working electrode.

In a particular embodiment there is provided an electrochemical sensor, wherein a sample may be actively moved through or over an oxygen scavenger in the sensing chamber and/or in the optional pre-chamber, and wherein there is further provided means for hindering a passage of oxygen (in the actively led sample) towards the working electrode, or even the sensing chamber membrane. In a specific exemplary embodiment, the pre-chamber is arranged for enabling a gas sample to be actively pumped through a solution comprising the aprotic solvent with the oxygen scavenger before it reaches the sensing chamber membrane, wherein the aprotic solvent with the oxygen scavenger hinders a passage of oxygen towards the working electrode or even the sensing chamber membrane. In another specific embodiment, the pre-chamber is arranged for enabling a sample to be actively pumped through a passageway in the pre-chamber, which passageway is bordered by an internal membrane within the pre-chamber, behind which internal membrane the oxygen scavenger is present, which oxygen scavenger enables removing oxygen from the actively led sample before it reaches the sensing chamber membrane.

It may in general be understood to be advantageous to hinder oxygen in passing to the working electrode, or even into the sensing chamber, since if the oxygen passes to the working electrode, it may interfere with the measurement of the analyte, since oxygen may pass electrons to the working electrode under the same conditions as the analyte passes electrons to the working electode. In other words, both the analyte and oxygen may cause a current to flow through the working electrode, and in case the current is caused by oxygen it may thus interfere with the measurement of the analyte.

By 'being arranged for hindering oxygen in passing from the associated volume to' a given element (such as working electrode or sensing chamber) may be understood, at least partially hindering. It it thus conceived that a certain amount of oxygen may reach the element, but that said amount is reduced with respect to what it would have been in the absence of the oxygen scavenger, such as reduced to a level of 90 % or less, such as 75 % or less, such as 50 % or less, such as 25 % or less, such as reduced to a level of 10 % or less, such as 5 % or less, such as 2 % or less, such as 1 % or less, such as 0.5 % or less, such as 0.1 % or less, such as 0.01 % or less, such as substantially zero %, such as zero %.

It may in general be preferred that little or no oxygen may be allowed to pass to the working electrode, such as 1 % or less, such as 0.5 % or less, such as 0.1 % or less, such as 0.01 % or less, such as substantially zero %, such as zero %.

It may be allowed in embodiments, that oxygen may pass into the sensing chamber. This may in particular be the case if the sensing chamber comprises an oxygen scavenger, such as an aprotic solvent comprising an oxygen scavnger to remove the entering oxygen before it reaches the working electrode.

Thus, aprotic solvent comprising an oxygen scavenger may be placed exclusively in the sensing chamber, exclusively in the pre-chamber or in both sensing chamber and pre-chamber, but in all cases little or no oxygen may be allowed to pass to the working electrode, such as 1 % or less, such as 0.5 % or less, such as 0.1 % or less, such as 0.01 % or less, such as substantially zero %, such as zero %.

The inventors have found that organophosphorus compounds are surprisingly advantageous in fulfilling the criteria mentioned above. Thus, a preferred embodiment of the present invention is an electrochemical sensor, wherein said oxygen scavenger is an organophosphorus compound. Phosphines have proved particularly useful and thus, in a preferred embodiment said oxygen scavenger is a phosphine.

In a preferred embodiment said phosphine is a compounds of formula (I), or salt thereof: 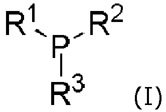 wherein R¹, R² and R³ independently are selected from the group consisting of hydrogen, optionally substituted alkyl, optionally substituted heteroalkyl, optionally substituted cycloalkyl, optionally substituted cycloheteroalkyl, optionally substituted aryl, and optionally substituted heteroaryl, with the proviso that at least one of R¹, R² and R³ is not hydrogen.

Preferably at least one of R¹, R² and R³ is hydrogen. Also, preferably at least one of R¹, R² and R³ is selected from the group consisting of optionally substituted alkyl, optionally substituted heteroalkyl, optionally substituted cycloalkyl, and optionally substituted cycloheteroalkyl. Furthermore, preferably at least one of R¹, R² and R³ is selected from the group consisting of an optionally substituted aryl, and optionally substituted heteroaryl.

Certain combinations of substituents on a phosphine may aid in fulfilling the criteria as described above in terms of e.g. solubility, oxygen reactivity and membrane tolerance. Thus, in a particularly preferred embodiment the phosphine of formula (I) is provided, wherein
- R¹ is hydrogen,
- R² is an optionally substituted alkyl, optionally substituted heteroalkyl, optionally substituted cycloalkyl or optionally substituted cycloheteroalkyl, and
- R³ is an optionally substituted aryl or optionally substituted heteroaryl.

For the present invention, said alkyls, cyclo- or heteroalkyls may preferably be C₁-C₂₀ alkyls, such as C₁₋₁₅, C₁-C₁₀, C₁-C₈ alkyls, such as preferably C₁-C₄ alkyls. Said aryls or heteroaryls may preferably be C₂-C₁₈ aryls or heteroaryls, such as C₂-C₁₆, C₂-C₁₄, C₂-C₁₂, C₂-C₁₀, C₂-C₈, such as preferably C₂-C₆ aryls or heteroaryls.

In a preferred embodiment said optionally substituted heteroalkyl is an optionally substituted heteroalkyl of formula (II):

-(CH₂)ₙ-Y-(CH₂)ₘ-Z-(CH₂)ₗCH₃ (II)

wherein n, m and I independently are integers from 0-10 and Y is a heteroatom and Z is a bond or a heteroatom, wherein said heteroatom is independently selected from NR⁴, O or S, wherein R⁴ is hydrogen or C₁-C₅ alkyl. An increased distance between the phosphine and a heteroatom in the heteroalkyl chain has been shown to affect the scavenging performance in a positive way, and thus n may preferably be 2-10, such as 3-10, such as 4-10, such as preferably 5-10.

In an advantageous embodiment the optional substituents of the present invention are ionic groups, which in combination with an organic or inorganic counterion form a salt. It has been found that such substituents help to reduce unwanted interaction between scavenger and certain membrane types, such as silicone membranes. Thus, a preferred embodiment is a phosphine of the present invention wherein said optionally substituted alkyl, optionally substituted heteroalkyl, optionally substituted cycloalkyl, optionally substituted cycloheteroalkyl, optionally substituted aryl, or optionally substituted heteroaryl comprises at least one ionic substituent and a counter-ion to form a salt.

Preferably, the ionic substituent is selected from the group consisting of ammonium and sulfonium, sulfonate and phosphonium. The counter-ion may be selected from the group consisting of anionic groups such as chloride, bromide, iodide, tetrafluorborate, hexafluorophosphate, tetraphenylborate, perchlorate, tetrakis(perfluoro-tert-butoxy)aluminate, trifluoroacetate, and thiocyanide, and cationic groups such as tetrabutylammonium, tetrabutylphosphonium, potassium, sodium, magnesium, and lithium.

A particularly preferred embodiment is therefore a phosphine of formula (I), wherein
- R¹ is hydrogen,
- R² is a substituted C₁-C₁₀ alkyl, comprising at least one ionic substituent and a counter-ion to form a salt.
- R³ is an optionally substituted C₆-C₁₄ aryl or optionally substituted C₂-C₁₀ heteroaryl.

The optionally substituted alkyl comprising at least one ionic substituent and a counter-ion to form a salt, may preferably be a group of formula (III): 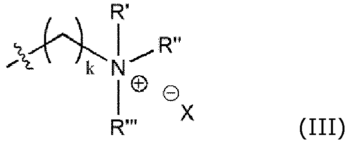 wherein k is an integer from 1-10, and
R', R" and R"' are independently selected from optionally substituted C₁-C₅ alkyl, optionally substituted aryl, optionally substituted heteroaryl, or any two of R', R" and R"' may be fused to form an optionally substituted cycloalkyl or heterocykloalkyl, and
X is a anionic counter-ion preferably selected from the group consisting of chloride, bromide, iodide, tetrafluorborate, hexafluorophosphate, tetraphenylborate, perchlorate, tetrakis(perfluoro-tert-butoxy)aluminate, thiocyanide, and trifluoroacetate.

The aryl or heteroaryl of the present invention may particularly be selected from the group consisting of phenyl, naphtyl, furanyl, pyrrolyl, imidazolyl, pyrazolyl, triazolyl, pyridinyl, pyrimidinyl, pyridazinyl, pyrazinyl, thiophenyl, oxazolyl, and thiazolyl, preferably phenyl.

The cycloalkyl or cycloheteroalkyl of the present invention may preferably be selected from the group consisting of cyclopropanyl, cyclobutanyl, cyclopentanyl, cyclohexanyl, tetrahydrofuranyl, tetrahydropyranyl, tetrahydrothiophenyl, tetrahydrothiopyranyl, piperidinyl, pyrrolidinyl, morpholinyl. Nitrogen containing heteroalkyls may preferably be N-alkyl substitiuted.

The optional substituents as described for R¹, R² and R³ and R', R" and R"' of the present invention may preferably be selected from the group consisting of C₁-C₅ alkyl, aryl, heteroaryl, hydroxy, amino, ammonium, sulfonium, sulfonate, phosphonium, halide, or cyano substituents. Preferably, said optional substituents are selected from the group consisting of hydroxyl, amine, ammonium, or sulfonate. The embodiments described earlier for alkyls and aryls also apply to the present substituents.

In a particularly preferred embodiment the phosphine of the present invention is selected from the group consisting of Diphenyl phosphine (Ph2PH), 2-(2-methoxyethoxy)ethylphosphin (MeO-PEG₂-PH), 2-(di-*tert-*butylphosphino)ethylamine (t-Bu₂PEtNH₂), 2-(di-*iso*-propylphosphino)ethylamine trifluoracetate (i-Pr₂PEtNH₃⁺CF₃COO⁻), 4-[(di-*tert-*butylphosphino)butyl]triethylammonium bromide (t-Bu₂PBuN(Et)₃⁺Br⁻), N-[4-(di-*tert*-butylphosphino)butyl]-N-methylmorpholinium bromide, tri-*n*-butylphosphine (*n*-Bu₃P).

The solvent or medium which incorporates the oxygen scavenger of the present invention must equally fulfil similar criteria to those valid for the oxygen scavenger, except that a scavenging effect on oxygen is not required. Preferred mediums include non-aqueous solvents and porous matrices. Porous matrices may be selected from the group consisting of polymers selected from the group consisting of silicone, PVC, polystyrene or latex, and also inorganic porous matrices selected from the group consisting of siliciumdioxide, zirconiumdioxide, titaniumdioxide, alumina, indiumnitride, or boronnitride.

A non-aqueous solvent should be essentially free of water. Preferably it is an aprotic solvent, i.e. without labile protons, which typically includes protons bound to heteroatoms. In a preferred embodiment the said aprotic solvent of the present invention is selected from the group consisting of acetonitrile, dimethylcarbonate, N,N-dimethylformamide (DMF), dimethylsulfoxide (DMSO), N-methylpyrrolidone, nitromethane, 1,1,3,3-tetramethylurea, perfluorodecaline and propylene carbonate, preferably propylene carbonate.

In an embodiment the electrochemical sensor is arranged so that when in use a path of the analyte when moving from the associated volume (106) to the working electrode (104) traverses the aprotic solvent comprising the oxygen scavenger. When the sensor is in use, then in order for an analyte (originally) situated in the associated volume to be measured, it travels along a path from the associated volume to the working electrode. Similarly, an oxygen species in the associated volume may travel along a similar path. However, in order to avoid oxygen interference at the working electrode, the sensor is arranged so that the path traverses the aprotic solvent comprising the oxygen scavenger. This may for example be carried out by placing the aprotic solvent comprising the oxygen scavenger in the sensing volume, such as having the electrolyte comprising the aprotic solvent comprising the oxygen scavenger, and/or by placing the aprotic solvent comprising the oxygen scavenger in the pre-chamber.

In an embodiment the electrolyte is comprising an aprotic solvent. In an embodiment the electrolyte, such as the electrolyte in the sensing chamber (120), comprises the oxygen scavenger, such as the electrolyte is comprising the aprotic solvent comprising the oxygen scavenger. A possible advantage of this may be that it enables oxygen scavenging within the sensing chamber. A possible advantage of this may be, that it enables rendering superfluous a pre-chamber comprising means for hindering oxygen in reaching the sensing chamber. A possible advantage of this may be that a faster and/or more sensitive sensor can be realized, since the pre-chamber may degrade performance as measured by those parameters. Another possible advantage of this may be that it enables a more simple sensor construction, since the pre-chamber may be dispensed with. In an embodiment, there is no pre-chamber. In an embodiment the sensing chamber membrane is in direct contact with the associated volume. Another possible advantage may be that it enables scavenging oxygen from the electrolyte compartment, redering superfluous a guard electrode. By 'the electrolyte is comprising an aprotic solvent' is understood that the working electrode (in the sensing chamber) is in physical contact with, such as immersed in, the aprotic solution with the oxygen scavenger.

In an embodiment there is presented an electrochemical sensor, further comprising:
- a pre-chamber (110) having a pre-chamber opening (112) towards the associated volume (106),
wherein the sensing chamber (120) is being placed adjacent the pre-chamber (110) or partially surrounded by the pre-chamber (110),
wherein the sensing chamber opening (122) is towards the pre-chamber (110).
wherein the sensing chamber opening membrane (124) is arranged so as to separate the sensing chamber volume (126) from a pre-chamber volume (116), said pre-chamber volume (116) being within the pre-chamber (110).

A possible advantage of having a pre-chamber, may in general be that it enables scavenging oxygen and or other species which are preferably kept out or in reduced concentration in the sensing chamber.

The sensing chamber is in embodiments understood to be placed adjacent the pre-chamber or partially surrounded by the pre-chamber, such as within the pre-chamber.

By 'pre-chamber' is understood a chamber, such as a casing, which delimits a pre-chamber volume volume within the pre-chamber from the surroundings external to the pre-chamber. However, it is encompassed by the present invention, that the pre-chamber may have one or more through-going holes in the delimiting walls, such as openings for electrical wiring or membranes. However, in general, the pre-chamber does not allow fluid passage from outside the pre-chamber to inside the pre-chamber. In particular embodiments, the pre-chamber may comprise walls made at least partially of glass. In particular embodiments, the pre-chamber may comprise walls made at least partially of a polymeric material, such as polyether ether ketone (PEEK).

By 'pre-chamber opening' is understood a through-going hole in the walls of the pre-chamber which through-going opening connects the associated volume with a pre-chamber volume within the pre-chamber.

It is understood, that 'adjacent' in this context is not to be construed as limiting to embodiments where the entire pre-chamber is in close contact, such as borders, the sensing chamber. In certain embodiments, the pre-chamber may comprise portions being distant from the sensing chamber.

It is understood, that 'adjacent' in this context is not to be construed as limiting to embodiments where the entire pre-chamber is in close contact, such as borders, the sensing chamber. In certain embodiments, the pre-chamber may comprise portions being distant from the sensing chamber. It may furthermore be understood, that the pre-chamber may be split into several pre-chamber sub-chambers, such as a pre-chamber sub-chamber being arranged for comprising a the aprotic solvent with the oxygen scavenger and another pre-chamber sub-chamber for ensuring passage of, e.g., a gas from the associated volume to the sensing chamber membrane. In such arrangement, the sample may come into contact with the oxygen scavenger through an internal membrane in the pre-chamber, which internal membrane separates the pre-chamber sub-chambers.

In an embodiment the aprotic solvent comprising the oxygen scavenger is at least partially comprised, such as fully comprised, within the pre-chamber (110).

By 'fully comprised' may be understood, that the aprotic solvent comprising the oxygen scavenger is exclusively placed in the pre-chamber, such as not in the sensing chamber, such as the working electrode not being in contact with the aprotic solution with the oxygen scavenger.

By 'partially comprised' may be understood, that at least some aprotic solvent comprising the oxygen scavenger is placed in the pre-chamber, but aprotic solvent comprising the oxygen scavenger may also be placed elsewhere (such as another portion of aprotic solvent comprising an oxygen scavenger), such as in the sensing chamber.

A possible advantage of having aprotic solvent comprising the oxygen scavenger in both pre-chamber and sensing chamber, may be that a concentration of oxygen scavenger may be kept relatively low. Another possible advantage may be that it may in turn enable a low zero current and better sensitivity (e.g., by enabling different dimensions (such as larger sensing chamber opening and/or pre-chamber opening), while still having a low amount of oxygen at the working electrode).

In an embodiment, the aprotic solvent comprising the oxygen scavenger is arranged for hindering oxygen in passing into the sensing chamber volume (126).

According to this embodiment, by the 'means for hindering oxygen in passing' is understood means which hinders oxygen in passing from the associated volume, such as the outside of the sensor, through the pre-chamber membrane, the pre-chamber volume and the sensing chamber membrane. It may thus be understood, that the means for hindering oxygen in passing enables keeping oxygen from the associated volume substantially out of the sensing chamber volume. A possible advantage of this may be that it enables having no oxygen scavenger in the sensing chamber.

In an embodiment the analyte is a gaseous analyte.

In an embodiment the analyte is any one of nitrous oxide (N₂O), carbon dioxide (CO₂), hydrogen (H₂), nitric oxide (NO), methane (CH₄), nitrogen (N₂), mono-nitrogen oxides (NOx) and/or hydrogen sulfide (H₂S).

In an embodiment the analyte is nitrous oxide (N₂O). By 'nitrous oxide (N₂O)' is understood a chemical compound being a molecule with the formula N₂O (N2O). Nitrous oxide may also be referred to as dinitric oxide or laughing gas.

In an embodiment the analyte is carbon dioxide (CO₂). By 'carbon dioxide (CO₂)' is understood a chemical compound being a molecule with the formula CO₂ (CO2).

In an embodiment the working electrode (104) comprises any one of: indium (In), platinum (Pt), gold (Au), silver (Ag), palladium (Pd), iridium (Ir) and/or carbon.

In an embodiment the working electrode (104) comprises indium (In). By 'the working electrode comprises indium (In)' is understood that the working electrode is arranged such that the catalytic properties of the element Indium (no. 49 in the periodic table) towards reduction of nitrous oxide are utilized. In an exemplary embodiment, indium is used as working electrode. In another embodiment, indium is deposited on the working electrode. Indium may be understood to be elemental indium. In another embodiment, indium may be understood to be, or comprise, indium, such as indium ions, such as indium oxide (In₂O₃).

In an embodiment the sensor comprises
- a pre-chamber (110) having a pre-chamber opening (112) towards the associated volume (106), where a pre-chamber membrane (114) is placed in the pre-chamber opening,
- a sensing chamber (120) being placed adjacent the pre-chamber (110) or partially surrounded by the pre-chamber (110), the sensing chamber (120) comprising,
   - A working electrode (104),
   - A reference electrode (108), and
   - An electrolyte,
      where the sensing chamber (120) has a sensing chamber opening (122) towards the pre-chamber (110), where a sensing chamber opening membrane (124) is placed in the sensing chamber opening (122),
wherein the pre-chamber membrane (114) is permeable to the analyte and is arranged so as to separate the associated volume (106) from a pre-chamber volume (116), said pre-chamber volume being within the pre-chamber (110), and wherein the sensing chamber opening membrane (124) is permeable to the analyte and is arranged so as to separate the pre-chamber volume (116) from a sensing chamber volume (126), said sensing chamber volume being within the sensing chamber (120),
where the pre-chamber (110) comprises an aprotic solvent comprising an oxygen scavenger,
wherein the aprotic solvent comprising the oxygen scavenger is at least partially comprised, such as fully comprised, within the pre-chamber (110), such as said aprotic solvent comprising said oxygen scavenger being arranged for hindering oxygen in passing into the sensing chamber volume (126).

By 'pre-chamber membrane' is understood a membrane material which is placed in the pre-chamber opening. The pre-chamber membrane is arranged so as to separate the associated volume from the pre-chamber volume within the pre-chamber. More specifically, the pre-chamber membrane is situated so as to fill the pre-chamber opening, so as to block passage from the associated volume to the pre-chamber volume of any substance incapable of penetrating through the pre-chamber membrane. It is understood, that the pre-chamber membrane may refer to a thin, film-like structure that separates two fluids, such as a liquid or gas in the associated volume, and a liquid or gas in the pre-chamber volume. However, it is also understood that the pre-chamber membrane may act as a selective barrier, allowing some particles or chemicals to pass through but not others. It may in particular be understood, that the pre-chamber membrane may be permeable to nitrous oxide and/or carbon dioxide. The pre-chamber membrane may in particular embodiment comprise, such as consist of, silicone, such as any one of the silicone sealants obtainable from Dow Corning with product number 732 or 734. A possible advantage of using silicone as pre-chamber membrane material may be that it combines good adhesion to glass with high permeability to, e.g, N₂O.

It may be understood, that in certain embodiments, the pre-chamber membrane may be dispensed with. It may be understood, that in some of these embodiments, there is provided means for keeping unwanted substances out of the pre-chamber volume, such as avoiding unwanted substances in reaching the sensing chamber membrane. In an exemplary embodiment, the pre-chamber is divided into sub-chambers, which performs various functions, such as one sub-chamber being a meander shaped pathway with reactive walls, such as membranes through which scavengers, such as oxygen scavengers, may diffuse, or through which the unwanted substances may diffuse. In such embodiment, an unwanted substance may enter partially into the pre-chamber, such as a pre-chamber sub-chamber, but may not reach the sensing chamber membrane, or may reach the sensing chamber membrane only in minute concentrations.

In an embodiment the sensing chamber membrane (124) and/or the pre-chamber membrane (114), is made of a gas permeable polymeric material.

In an embodiment the sensing chamber membrane (124) and/or the pre-chamber membrane (114), is made of a material chosen from the group consisting of: teflon (teflon may be used interchangeably with polytetrafluoroetylen (PTFE)), polysiloxanes (silicones), polyphosphazanes, nitrocellulose, polyacrylonitrile.

In an embodiment the sensing chamber membrane (124), is made of teflon. In an embodiment the pre-chamber membrane (114), is made of teflon.
A possible advantage of teflon, may be that it may be compatible with certain solvents and oxygen scavengers.

In an embodiment the sensor response and/or the baseline signal is is substantially stable over extended periods of time.

In an embodiment the electrolyte comprises propylene carbonate.

In another embodiment there is provided an electrochemical sensor, the sensor response (sensitivity) and/or the baseline signal is substantially stable over extended periods of time, such as substantially stable over 30 days, such as 60 days, such as 90 days. By substantially stable may be understood that the baseline signal may be within ±100 % of an initial value, such as within ±50 % of an initial value. By substantially stable may be understood that the response signal (sensitivity, such as sensitivity towards 80 µM N₂O) may be within ±100 % of an initial value, such as within ±50 % of an initial value, such as within ±25 % of an initial value, such as within ±10 % of an initial value. In a specific embodiment, the response signal may be within ±25 % of an initial value for at least 90 days.

In another embodiment there is provided an electrochemical sensor, wherein the aprotic solvent, such as the aprotic solvent in the electrolyte and or the aprotic solvent with the oxygen scavenger, is propylene carbonate. An advantage of propylene carbonate (PC) may be that it enables dissolving ionic compounds, such as compounds comprising tetrabutylammonium, such as TBA-I, TBA-Cl, TBA-BF₄ or TBA-ClO₄. Another possible advantage may be that PC does not degrade commonly used membrane materials, such as silicone. Another possible advantage is that it is non-toxic.

In the context of the present invention "oxygen scavenger" is defined in the broadest sense as any compound capable of removing oxygen molecules from a medium such as a solvent or porous matrix. Advantageous oxygen scavengers are soluble and/or dispersible in a given preferred medium. Oxygen scavengers are often reducing agents or agents who form chemical bonds with oxygen atoms by other means. For example, an 'oxygen scavenger' may be a chemical substance which may be able to hinder a passing of oxygen, such as diffusion of oxygen, by removing or absorbing oxygen.

The oxygen scavenger of the present invention must fulfil a number of criteria to perform well in the sensor of the present invention. Thus, the oxygen scavenger should have good solubility in the solvent or medium used in the sensor, it should provide for highly effective removal of oxygen, e.g. from the solvent solution used while allowing analyte (e.g. N₂O) diffusion to the sensor. Furthermore, it must be compatible with the materials used in the sensor, particularly the membrane materials, which come into contact with the oxygen scavenger. Finally, it must also be considered that both the scavenger itself and the products obtained after the scavenger has reacted with oxygen should preferably stay in solution or at least not precipitate to a degree which changes the rate of diffusion of analytes towards the working electrode, blocks membranes very rapidly or otherwise degrades the sensor capabilities.

In specific embodiements, where the pre-chamber comprises a freely diffusing oxygen scavenger, the pre-chamber may be arranged so that a diffusion of the oxygen scavenger into a volume in front of the sensing chamber membrane (from the remaining portion of the pre-chamber volume) may at least be large enough to balance the consumption of the oxygen scavenger in the region in front of the sensing chamber membrane. In specific embodiments, the volume in front of the sensing chamber membrane may correspond to a volume between the pre-chamber membrane and the sensing chamber membrane, and the remaining portion of the pre-chamber volume, which may be referred to as a "bulk" volume, may be large enough in order to effectively act as a reservoir for the oxygen scavenger.

In a specific embodiment, the diameter of the sensing chamber membrane should be as large as possible (to capture all incoming analyte) without compromising a necessary rate of diffusion of fresh oxygen scavenger to a path of analytes and oxygen from the associated volume to the working electrode.

In another embodiment, such as an embodiment where the analyte is nitrous oxide, there is provided an electrochemical sensor, wherein an ionic compound is dissolved in the electrolyte, the ionic compound comprising tetrabutylammonium (TBA). An advantage of using TBA may be, that it has proven to be able to provide promising results in terms of sensor performance. In another embodiment there is provided an electrochemical sensor, wherein an ionic compound is dissolved in the electrolyte, the ionic compound being chosen from the group comprising
- tetrabutylammonium iodide (TBA-I),
- tetrabutylammonium chlorate (TBA-Cl),
- tetrabutylammonium flouroborate (TBA-BF₄) and/or tetrabutylammonium perchlorate (TBA-ClO₄).

In another embodiment there is provided an electrochemical sensor, wherein the sensor is suited for measuring in any one of: a gas, a liquid. By a gas may be understood a substantially pure gas, such as a pure gas, or there may be understood a gas mixture, such as air.

In another embodiment there is provided an electrochemical sensor further comprising
- a voltage generating means, such as a potentiostat, for holding the working electrode at a first voltage with respect to the reference electrode, such that nitrous oxide may be reduced at the working electrode causing a current to flow through the electrode,
- a current sensing means, such as an ammeter, for sensing the presence of the current, such as quantifying the current.

In another embodiment there is provided an electrochemical sensor, wherein the sensor is arranged for providing real-time measurements, such as by providing a sensor of sufficiently small dimensions, such as enabling response time of less than 1 hr, such as less than 30 minutes, such as less than 15 minutes, such as less than 10 minutes, such as less than 5 minutes, such as less than 2 minutes, such as less than 90 seconds, 60 seconds, such as within 10-60 seconds. The response time is understood to be the period of time it takes before the response reaches 90 % of the new equilibrium signal.

In another embodiment there is provided an electrochemical sensor, wherein the sensor further comprises a third electrode working as a counter electrode. 'Counter electrode' is known in the art and understood as an electrode which can deliver or receive electrons (i.e., current) from the working electrode. The counter electrode may also be referred to as an auxiliary electrode. An advantage of such three-electrode setup may be that less current needs to be drawn from or to the reference electrode.

In a second aspect, the invention further relates to a method (440) for electrochemically sensing an analyte in an associated volume, the method comprising
- providing (s444) a sensing chamber, the sensing chamber comprising,
   ▪ A working electrode (104),
   ▪ A reference electrode (108), and
   ▪ An electrolyte,
   where the sensing chamber has a sensing chamber opening (122), where a sensing chamber opening membrane is placed in the sensing chamber opening,
wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
wherein
the electrochemical sensor (100) comprises an aprotic solvent comprising an oxygen scavenger, said aprotic solvent comprising said oxygen scavenger being arranged for hindering oxygen in passing from the associated volume (106) to the working electrode (104), the method further comprising reducing or oxidizing (s446) the analyte at the working electrode and measuring (s448) the corresponding current flowing through the working electrode.

In an embodiment, the method comprises:
- providing (s442) a pre-chamber having a pre-chamber opening towards the associated volume, where a pre-chamber membrane is placed the pre-chamber opening,
- providing (s444) a sensing chamber being placed adjacent the pre-chamber or partially surrounded by the pre-chamber, the sensing chamber comprising,
   ▪ A working electrode (104),
   ▪ A reference electrode (108), and
   ▪ An electrolyte,
      where the sensing chamber has a sensing chamber opening towards the pre-chamber, where a sensing chamber opening membrane is placed in the sensing chamber opening,
   wherein the pre-chamber membrane (114) is permeable to the analyte and is arranged so as to separate the associated volume (106) from a pre-chamber volume (116), said pre-chamber volume being within the pre-chamber (110), and
   wherein the sensing chamber opening membrane (124) is permeable to the analytes and is arranged so as to separate the pre-chamber volume (116) from a sensing chamber volume (126), said sensing chamber volume being
   within the sensing chamber (120),
where the pre-chamber (110) comprises an aprotic solvent comprising an oxygen scavenger, said aprotic solvent comprising said oxygen scavenger being arranged for hindering oxygen in passing from the associated volume (106) to the working electrode (104), such as for hindering oxygen in passing into the sensing chamber volume (126), the method further comprising reducing or oxidizing (s446) the analyte at the working electrode and measuring (s448) the corresponding current flowing through the working electrode.

In a third aspect, the invention further relates to use of an electrochemical sensor (100) according to the first aspect for sensing the presence of, such as measuring quantitatively, an analyte in an associated volume, such as the associated volume being occupied by wastewater, sea water, drinking water, gas emission from soil as earth surface or surface of a field. This aspect of the invention is particularly, but not exclusively, advantageous in that the sensor may advantageously be used for a large number of applications, such as environmental monitoring, such as biological research, such as sensing an analyte, such as nitrous oxide, in wastewater.

The first, second and third aspect of the present invention may each be combined with any of the other aspects. These and other aspects of the invention will be apparent from and elucidated with reference to the embodiments described hereinafter.

### BRIEF DESCRIPTION OF THE FIGURES

The first, second and third aspect according to the invention will now be described in more detail with regard to the accompanying figures. The figures show one way of implementing the present invention and is not to be construed as being limiting to other possible embodiments falling within the scope of the attached claim set.
Figure 1 is a schematic showing the structure of a sensor.
Figure 2 is a schematic showing the structure of another sensor.
Figure 3 is a flow-chart of a method according to the invention.
Fig. 4 shows microscopic images of a sensor tip.
Fig. 5 shows stability of the measuring characteristics during a 90 days period.
Fig. 6 shows calibration examples for an N₂O sensor performed 50 days apart.
Fig. 7 shows a microscopic image of a 1-compartment N₂O.
Fig. 8 shows a response test to 100 µM N₂O on day 1 for a 1-compartment sensor.
Fig. 9 shows microscopic images showing three examples of different designs for CO₂ sensors.
Fig. 10 shows calibration test for a 2-compartment CO₂ sensor.
Fig. 11 shows calibration test for a 1-compartment CO₂ sensor.
Figs. 12-13 shows exemplary oxygen depth.
Fig. 14 shows shows microscopic images of a sensor tip.

### DETAILED DESCRIPTION OF AN EMBODIMENT

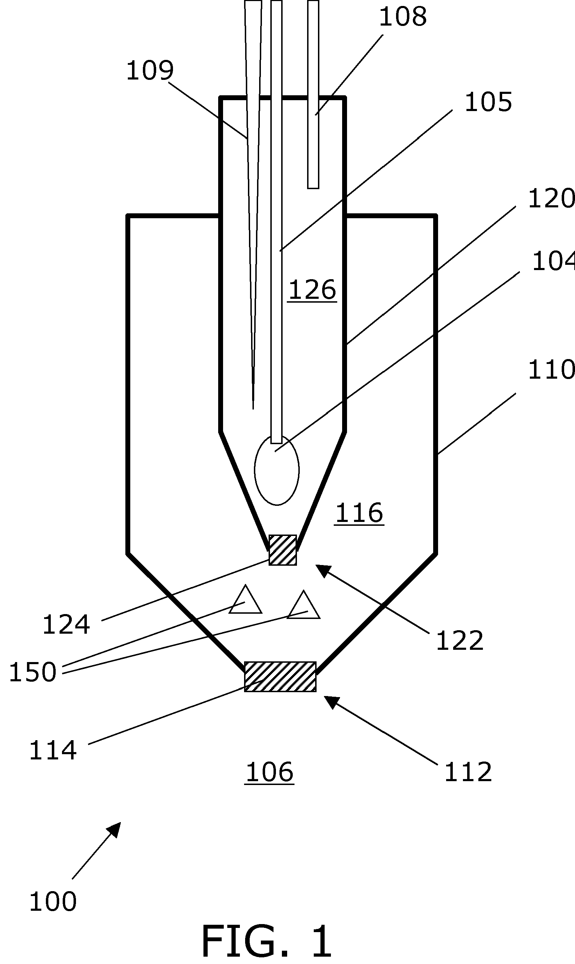

FIG 1 shows an electrochemical sensor (100) for sensing an analyte, said analyte being nitrous oxide, in an associated volume (106), the sensor comprising
- a sensing chamber (120), the sensing chamber (120) comprising,
   - A working electrode (104),
   - A reference electrode (108), which in the present embodiment comprises silver (Ag), and
   - An electrolyte, which in the present embodiment is comprising an aprotic solvent, which in the present embodiment is propylene carbonate (PC), where it is understood that electrolyte is comprising an aprotic solvent wherein an ionic compound, which in the present embodiment is tetrabutylammonium-iodide (TBA-I), is dissolved, and where the electrolyte electrically connects the reference electrode, the working electrode and the guard electrode 109,
   where the sensing chamber (120) has a sensing chamber opening (122), where a sensing chamber opening membrane (124) is placed in the sensing chamber opening (122),
wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
wherein
the electrochemical sensor (100) comprises an aprotic solvent comprising an oxygen scavenger, said aprotic solvent comprising said oxygen scavenger being arranged for hindering oxygen in passing from the associated volume to the working electrode (104),
and said sensor further comprising:
- a pre-chamber (110) having a pre-chamber opening (112) towards the associated volume (106),
wherein the sensing chamber (120) is being placed adjacent the pre-chamber (110) and partially surrounded by the pre-chamber (110), such as the sensing chamber being enclosed by the pre-chamber around, such as 360 degrees around a longitudinal axis of the sensing chamber,
wherein the sensing chamber opening (122) is towards the pre-chamber (110) and
wherein the sensing chamber opening membrane (124) is arranged so as to separate the sensing chamber volume (126) from a pre-chamber volume (116), said pre-chamber volume (116) being within the pre-chamber (110),
and
wherein the aprotic solvent comprising the oxygen scavenger is at least partially comprised, and in the present embodiment as fully comprised, within the pre-chamber (110),
and
where the aprotic solvent comprising the oxygen scavenger is arranged for hindering oxygen in passing into the sensing chamber volume (126).

The working electrode 104 is electrically connected to auxiliary equipment, such as a potentiostat, via an electrical conductor 105, which may be a glass-insulated platinum wire. The sensing chamber membrane is silicone, the pre-chamber membrane is teflon, the aprotic solvent with oxygen scavenger is 1 M di-phenylphoshine in propylene carbonate.

In the exemplary embodiment shown in FIG 1, the aprotic solvent is propylene carbonate, the oxygen scavenger (150) comprises di-phenylphospine, and the ionic compound is tetrabutylammonium iodide (TBA-I). Furthermore, the pre-chamber volume is approximately 5 mL. The associated volume in this specific, exemplary embodiment comprises waste water, but it is noted that it could also have been another liquid or gas comprising N₂O.

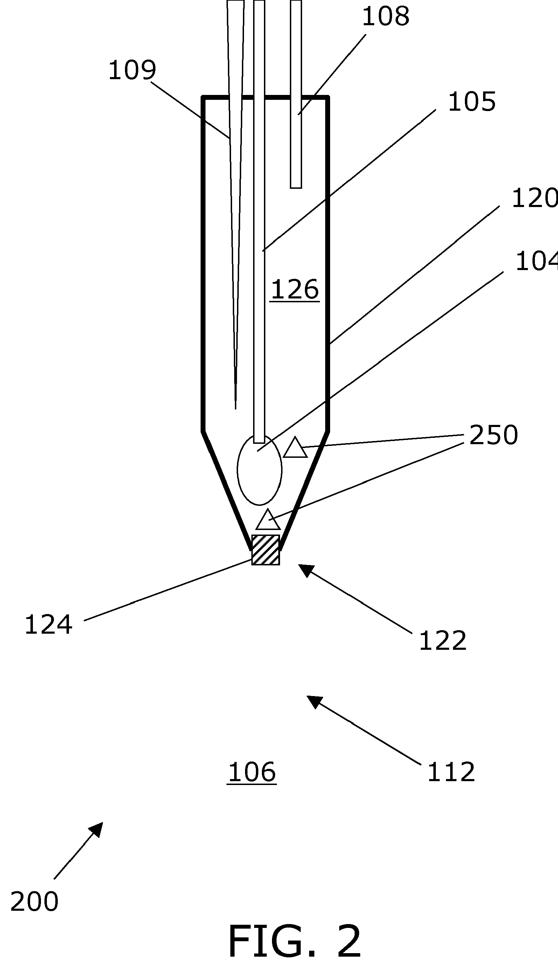

FIG 2 shows an electrochemical sensor (200) for sensing an analyte, said analyte being nitrous oxide, in an associated volume (106), the sensor comprising
- a sensing chamber (120), the sensing chamber (120) comprising,
   - A working electrode (104),
   - A reference electrode (108), and
   - An electrolyte,
   where the sensing chamber (120) has a sensing chamber opening (122), where a sensing chamber opening membrane (124) is placed in the sensing chamber opening (122),
wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
wherein
the electrochemical sensor (100) comprises an aprotic solvent comprising an oxygen scavenger, said aprotic solvent comprising said oxygen scavenger being arranged for hindering oxygen in passing from the
associated volume to the working electrode (104),
and
wherein the electrolyte, such as the electrolyte in the sensing chamber (120), comprises the oxygen scavenger, such as wherein the electrolyte is comprising the aprotic solvent comprising the oxygen scavenger (250).

The sensor (200) is similar to the sensor (100) of Fig. 1, except that the sensor (200) has no pre-chamber and has the aprotic solvent with the oxygen scavenger (250) in the sensing chamber. In the examplary embodiment, the sensing chamber membrane is a silicone membrane, the electrolyte, which is also the aprotic solvent with oxygen scavenger, comprises as oxygen scavenger 0.5 M di-phenylphosphine in the electrolyte. The aprotic solvent (electrolyte) is propylene carbonate with 0.3 M tetrabutylammonium iodide.

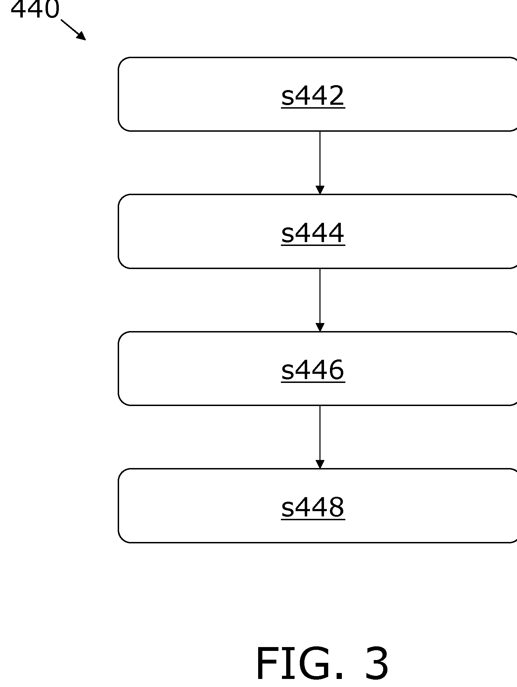

FIG 3 shows a method (440) for electrochemically sensing an analyte in an associated volume, the method comprising
- providing (s444) a sensing chamber, the sensing chamber comprising,
   ▪ A working electrode (104),
   ▪ A reference electrode (108), and
   ▪ An electrolyte,
   where the sensing chamber has a sensing chamber opening (122), where a sensing chamber opening membrane is placed in the sensing chamber opening,
wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
wherein the electrochemical sensor (100) comprises an aprotic solvent comprising an oxygen scavenger, said aprotic solvent comprising said oxygen scavenger being arranged for hindering oxygen in passing from the associated volume (106) to the working electrode (104), the method further comprising reducing or oxidizing (s446) the analyte at the working electrode and measuring (s448) the corresponding current flowing through the working electrode.

### EXAMPLES

### Design of sensors:

The general design of the N₂O sensor used during these tests comprised a 75 - 90 µm N₂O transducer with an inserted 150 µm silicone membrane as the sensing chamber membrane and an optional pre-chamber with a 70-100 µm wide and an about 100 µm long silicone membrane (see examples, Fig. 1). Alternatively, the pre-chamber was removed or the pre-chamber membrane was a 12.5 µm long teflon membrane.

### Example 1

Sensor with 1 M di-phenylphosphine in pre-chamber and silicone membrane as sensing chamber membrane and teflon membrane (12.5 µM) as pre-chamber membrane. The aprotic solvent with oxygen scavenger is propylene carbonate.

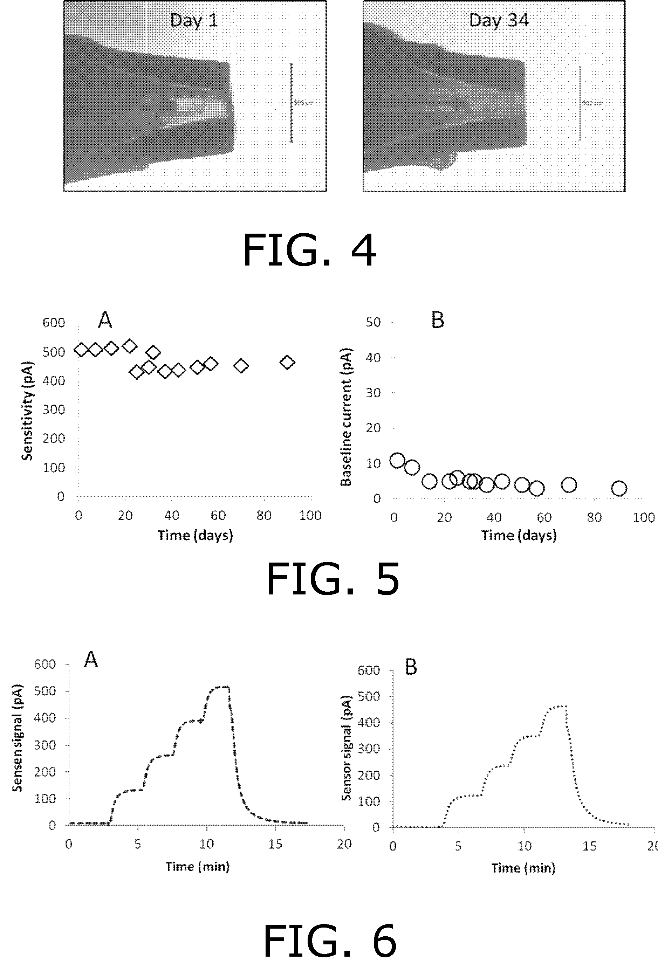

Fig. 4 shows microscopic images of the sensor tip on days 1 (left) and 34 (right) showing the internal N₂O transducer with a standard 150 µm silicone membrane and a pre-chamber with a 600 µm tip where the opening is covered by a 12.5 µm Teflon sheet. Both scale bars are 500 µm.

Fig. 5 shows that this sensor design is not showing a significant change in the measuring characteristics with a gradual loss of analytical sensitivity or an increasing baseline level, which suggests that the Teflon membrane is unaffected by the phosphine as also indicated from microscopic inspections of the sensor tip (Fig. 4).

More particularly, Fig. 5 shows stability of the measuring characteristics during a 90 days period for a sensor constructed with 1 M diphenylphosphine solution in the pre-chamber and pre-chamber membrane being a 12.5 µm Teflon membrane. The figures show developments in the sensitivity towards 80 µM N₂O (A) and baseline current (B).

Fig. 6 shows two calibration examples performed 50 days apart for an N₂O sensor designed with a 1M di-phenylphosphine solution in the pre-chamber and a pre-chamber Teflon membrane showing the continuous sensor signal at stepwise additions of 20 µM N₂O on days 7 (A) and 57 (B).

The sensor according to this example be particularly advantageous, due to: High sensitivity towards N2O, low and stable baseline level, no oxygen interference, stable performance, no apparent membrane effects.

### Example 2

1-compartment sensor, i.e., sensor without pre-chamber with 0.5 M di-phenylphosphine in electrolyte and 1 x silicone membrane as the sensing chamber membrane. The aprotic electrolyte is propylene carbonate with 0.3 M Tetrabutylammonium iodide.

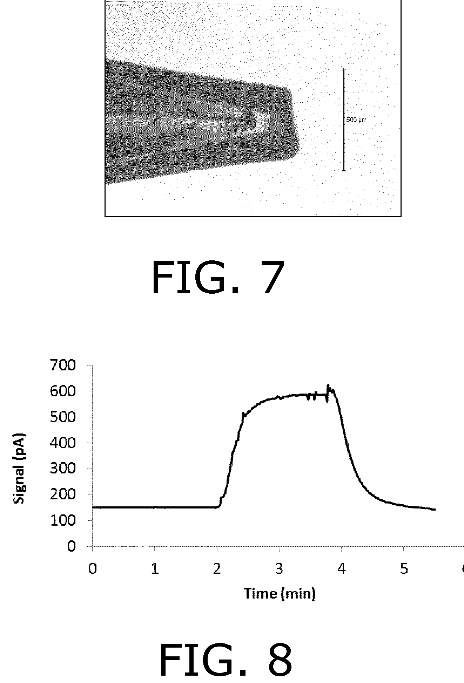

Construction of a functional one compartment (only sensing chamber, no pre-chamber) N₂O sensor was tested through the use of a sensor electrolyte that contained 0.5 M di-phenylphosphine and a retracted cathode position (see Fig. 7). The high internal concentration of phosphine was surprisingly not found to affect the sensor baseline, and the sensor was demonstrating a high sensitivity towards N₂O.

Fig. 7 shows a microscopic image of a 1-compartment (only sensing chamber, no pre-chamber) N₂O sensor with 0.5 M di-phenylphosphine inside the electrolyte and a 50 µm retracted cathode from the membrane. The scale bar is 500 µm.

### Example 3

Oxygen scavenger being 2-(di-*tert*-butylphosphino)ethylamine. The aprotic solvent with oxygen scavenger is propylene carbonate.

The sensor is showing a high response to N₂O and a fast response time (Fig. 8).

Fig. 8 shows a response test to 100 µM N₂O on day 1 for a sensor prepared with an aprotic solvent in the pre-chamber containing 1M 2-(di-*tert-*butylphosphino)ethylamine.

### Example 4

Di-phenylphosphine has been evaluated for the development of a CO₂ sensor based on a phosphine oxygen scavenger.

Design of sensors: The CO₂ sensors were prepared with a gold plated cathode and contained an aprotic electrolyte comprising Tetrabutylammonium iodide (TBAI) in propylene carbonate (PC). The aprotic solvent with oxygen scavenger is propylene carbonate.

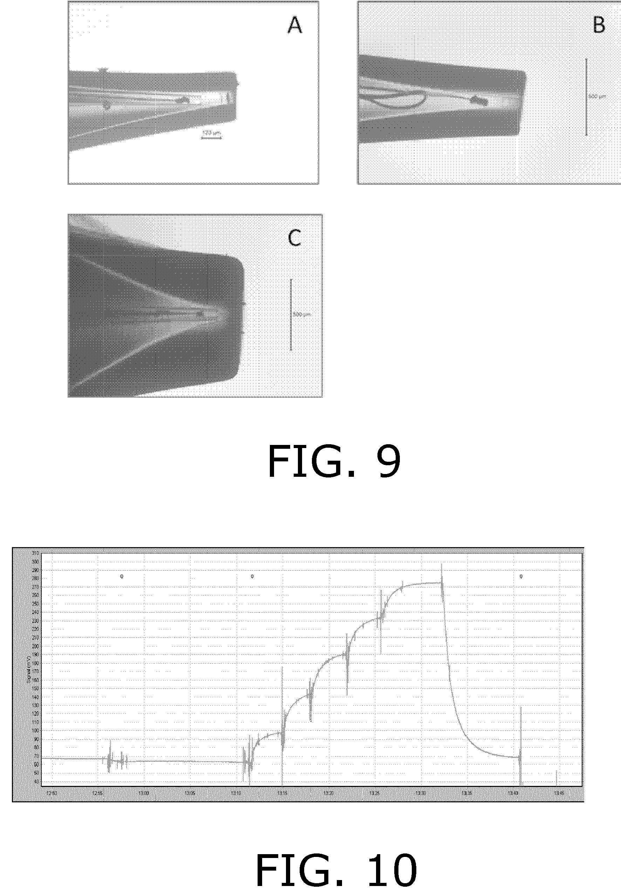

A number of different designs have been tested (Fig. 9):
- 2-compartment sensor (sensing chamber and pre-chamber) with two silicone membranes (both sensing chamber membrane and pre-chamber membrane).
- 1-compartment sensor (without pre-chamber) with silicone membrane and phosphine in the electrolyte.
- 2-compartment sensor with silicone membrane as the sensing chamber membrane and Teflon membrane as pre-chamber membrane.

Fig. 9 shows microscopic images with three examples of different designs for CO₂ sensors prepared with di-phenylphosphine: (A) 2-compartment sensor with 2 x silicone membrane (B) 1-compartment sensor with silicone membrane, and (C) 2-compartment sensor with silicone membrane and Teflon membrane. The pictures were taken just after the addition of the phosphine to the sensor, and the scale bars are (A) 123 µm, (B) 500 µm and (C) 500 µm.

### Results:

The results that were obtained for the different CO₂ sensors were similar, but with some variation in measuring characteristics (i.e. sensitivity and response time) due to variation in sensor size and design.

Overall the results demonstrate that phosphine can be used to produce highly sensitive CO₂ sensors with apparent linear response characteristics (Figs. 10-11), as it allows CO₂ to pass through aprotic solution comprising an oxygen scavenger. Summary of performance characteristics: High sensitivity towards CO₂ (up to 1000 pA/mM) but high baseline level and unstable measuring properties.

### Calibration examples:

Fig. 10 shows calibration test for a 2-compartment CO₂ sensor designed with a 1 M di-phenylphosphine solution in the pre-chamber and two silicone membranes (both sensing chamber membrane and pre-chamber membrane) showing the continuous sensor signal at stepwise additions of 0.2 mM CO₂ on day 1. The horizontal axis time scale spans a period from 12:50 to 13:45. The vertical axis spans a millivolt signal from 40 mV to 310 mV.

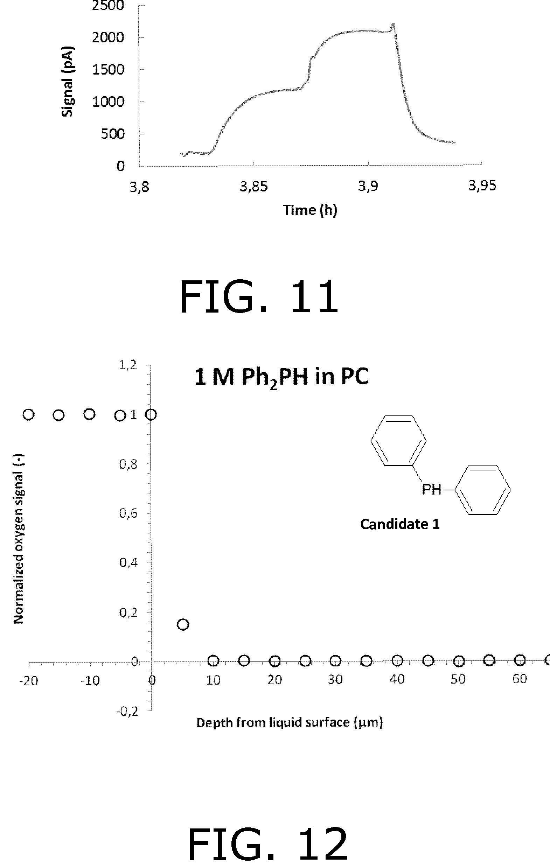

Fig. 11 shows calibration test for a 1-compartment CO₂ sensor designed with a 1 M di-phenylphosphine solution in the pre-chamber and one silicone membrane showing the continuous sensor signal at stepwise additions of 1 mM CO₂ on day 1.

The electrolyte used for testing the performance of the electrochemical sensor comprises an aprotic solvent and an ionic compound therein dissolved, so as to electrochemically connect the reference electrode (RE) and the working electrode (WE) used.

Since the metal present in the working electrode is sensitive to hydrogen element reduction to hydrogen gas, the use of an aprotic solvent has the main advantage of avoiding this side reaction and thus enhancing the signal to noise ratio towards the detection of the desired species.

### Example 5

### Solubility test

The solubility of the oxygen scavengers were determined by addition of a volume of propylene carbonate, such that the final concentration of a fully dissolved candidate will be 1 M. In some cases, addition of methanol is needed to achieve full dissolution. In other cases, solubility tests with acetonitrile instead of propylene carbonate was performed.

In the case that solids remained after addition of solvent and vigorous stirring, the solubility of a candidate is assessed by visual inspection of the amount of remaining compound versus the amount of compound prior to solvent addition.
In the case of a commercially available candidate dissolved in tetrahydrofuran (THF), the THF is removed under reduced pressure prior to addition of solvent.

### Profiling

The penetration of oxygen through the solution of the candidates was determined using an OX10 oxygen microsensor from Unisense A/S in conjunction with a microprofiling laboratory stand.

A small capillary, cautiously filled with candidate solution in order to avoid bulk oxidation, was placed underneath the tip of the oxygen microsensor. The microprofiling stand was remotely controlled by the bundled software in order to move the microelectrode tip with five to ten micrometers between each concentration measurement. The microelectrode was calibrated prior to each candidate measurement using air-bubbled demineralised water and oxygen-free alkaline ascorbic acid solution.

The microsensor was used to profile oxygen-free alkaline ascorbic acid solution prior to and after measurement of each candidate to 1) ensure that physical damage during candidate measurement could be detected and to 2) provide baseline signal for normalisation of candidate profiles.

Each candidate profile was subtracted the baseline signal and normalised with respect to surface oxygen concentration in order to assess the candidate oxygen removal efficiency. A normalised signal of unity equals surface oxygen concentration and a normalised signal of 0.01 equals a dissolved oxygen concentration of 1 % of surface concentration.

### Sample storage and handling

All samples of the candidate solutions were stored under dry argon in septum-capped (butyl rubber) vials, until the samples could be transferred into nitrous oxide sensors.

The liquid samples withdrawn for use in nitrous oxide sensors were quickly transferred by syringe technique from the septum-capped vials into the nitrous oxide sensors.

**Summary (Table I)**

| Candidate | Solvent^{†} | Solubility | Oxygen profile depth^{‡} |
|---|---|---|---|
| 1 | PC | ≥ 1 M | 7 µm |
| 2 | PC | ≥ 1 M | 260 µm |
| 4 | PC | ≥ 1 M | 7 µm |
| 8 | PC | ≥ 1 M | 75 µm |
| 9 | 10 % MeOH in PC | ≥ 1 M | 17 µm |
| 10 | PC or MeCN | Slightly | 47 µm |
| 12 | PC | ≥ 1 M | 37 µm |

| | | | |
|---|---|---|---|
| †: PC = Propylene carbonate, MeOH = methanol, MeCN = acetonitrile. ‡: Defined as distance from liquid surface where oxygen concentration is 1 % of oxygen concentration at liquid surface. | | | |

### TABLE I

### List of candidates:

All candiates dissolved in propylene carbonat in a concentration of 1 M, unless otherwise stated.
Candidate 1: Diphenylphosphine (Ph₂PH) 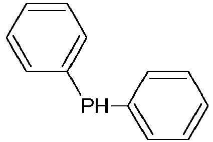 For 0.1 M solution: Oxygen profile depth ca. 75 µm.
Candidate 2: 2-(2-methoxyethoxy)ethylphosphin (MeO-PEG2-PH) 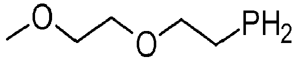
Candidate 4: 2-(di-*tert*-butylphosphino)ethylamine (*t*-Bu₂PEtNH₂) 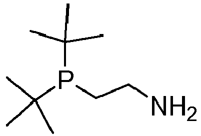
Candidate 8: 2-(di-*iso-*propylphosphino)ethylamine trifluoracetate (i-Pr₂PEtNH₃⁺CF₃COO⁻) 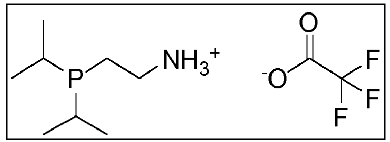
Candidate 9:
   [4-(di-*tert*-butylphosphino)butyl]triethylammonium bromide (*t*-Bu₂PBuN(Et)₃⁺Br⁻) 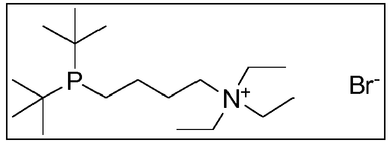
Candidate 10:
   *N*-[4-(di-*tert*-butylphosphino)butyl]-*N*-methylmorpholinium bromide 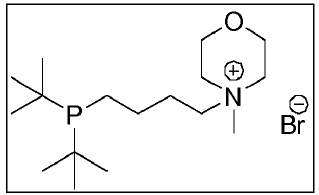 Concentrationen in propylene carbonate (PC) substantially lower than 1 M.
Candidate 12:

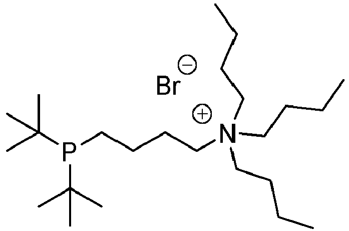

Figs. 12-13 shows exemplary oxygen depth profiles for candidates 1 and 8.

### Example 6

Sensor with both sensing chamber and pre-chamber and 1 M di-phenylphosphine in pre-chamber and silicone membrane as sensing chamber membrane and silicone membrane as pre-chamber membrane. The aprotic solvent with oxygen scavenger is propylene carbonate.

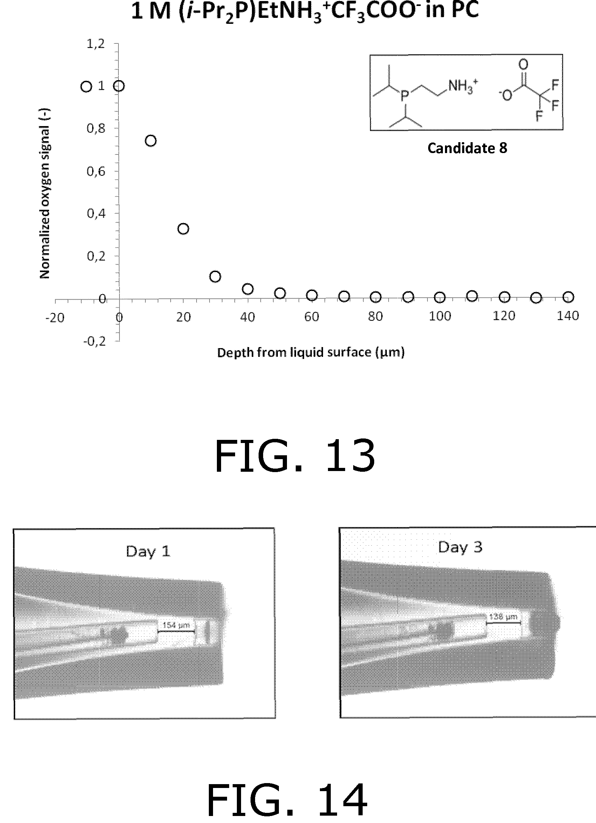

Fig. 14 shows microscopic images of the sensor tip on days 1 (left, with a scale bar of 154 µm) and 3 (right, with a scale bar of 138 µm).

To sum up, an electrochemical sensor (100) for sensing an analyte in an associated volume (106), the sensor comprising a sensing chamber (120), the sensing chamber (120) comprising, a working electrode (104), wherein the electrochemical sensor (100) comprises an aprotic solvent comprising an oxygen scavenger, said aprotic solvent comprising said oxygen scavenger being arranged for hindering oxygen in passing from the associated volume to the working electrode (104). In a particular embodiment the oxygen scavenger is a phosphine.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

In alternative embodiments E1-E50, there is presented:
E1.An electrochemical sensor (100) for sensing an analyte in an associated volume (106), the sensor comprising
   - a sensing chamber (120), the sensing chamber (120) comprising,
      - A working electrode (104),
      - A reference electrode (108), and
      - An electrolyte,
      where the sensing chamber (120) has a sensing chamber opening (122), where a sensing chamber opening membrane (124) is placed in the sensing chamber opening (122),
   wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
   wherein
   the electrochemical sensor (100) comprises an aprotic solvent comprising an oxygen scavenger.
E2.An electrochemical sensor according to any of the preceding embodiments, wherein said aprotic solvent comprising said oxygen scavenger being arranged for hindering oxygen in passing from the associated volume to the working electrode (104), such as enabling that an oxygen concentration at the working electrode with respect to an oxygen concentration in the associated volume is less than 10 %, such as less than 1 %, such as less than 0.1 %, such as substantially 0 %, such as 0 %.
E3.An electrochemical sensor according to any of the preceding embodiments, wherein said oxygen scavenger is an organophosphorus compound.
E4.An electrochemical sensor according to any of the preceding embodiments, wherein said oxygen scavenger is a phosphine.
E5.An electrochemical sensor according to embodiment 3, wherein said phosphine is a compound of formula (I), or salt thereof: 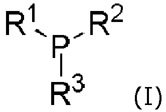 wherein R¹, R² and R³ independently are selected from the group consisting of hydrogen, optionally substituted alkyl, optionally substituted heteroalkyl, optionally substituted cycloalkyl, optionally substituted cycloheteroalkyl, optionally substituted aryl, and optionally substituted heteroaryl, with the proviso that at least one of R¹, R² and R³ is not hydrogen.
E6.An electrochemical sensor according to embodiment E5, wherein at least one of R¹, R² and R³ is hydrogen.
E7.An electrochemical sensor according to any one of embodiments E5-E6, wherein at least one of R¹, R² and R³ is selected from the group consisting of optionally substituted alkyl, optionally substituted heteroalkyl, optionally substituted cycloalkyl, and optionally substituted cycloheteroalkyl.
E8.An electrochemical sensor according to any one of embodiments E5-E7, wherein at least one of R¹, R² and R³ is selected from the group consisting of an optionally substituted aryl, and optionally substituted heteroaryl.
E9.An electrochemical sensor according to embodiment E5, wherein
   - R¹ is hydrogen,
   - R² is an optionally substituted alkyl, optionally substituted heteroalkyl, optionally substituted cycloalkyl or optionally substituted cycloheteroalkyl, and
   - R³ is an optionally substituted aryl or optionally substituted heteroaryl.
E10. An electrochemical sensor according to any one of embodiments E5-E9, wherein said alkyls, cyclo- or heteroalkyls are C₁-C₂₀ alkyls, such as C₁₋₁₅, C₁-C₁₀, C₁-C₈ alkyls, such as preferably C₁-C₄ alkyls.
E11. An electrochemical sensor according to any one of embodiments E5-E10, wherein said aryls or heteroaryls are C₂-C₁₈ aryls or heteroaryls, such as C₂-C₁₆, C₂-C₁₄, C₂-C₁₂, C₂-C₁₀, C₂-C₈, such as preferably C₂-C₆ aryls or heteroaryls.
E12. An electrochemical sensor according to any one of embodiments E5-E11, wherein said optionally substituted heteroalkyl is an optionally substituted heteroalkyl of formula (II):

   -(CH₂)ₙ-Y-(CH₂)ₘ-Z-(CH₂)ₗCH₃ (II)

   wherein n, m and I independently are integers from 0-10 and Y is a heteroatom and Z is a bond or a heteroatom, wherein said heteroatom is independently selected from NR⁴, O or S, wherein R⁴ is hydrogen or C₁-C₅ alkyl.
E13. An electrochemical sensor according to embodiment E12, wherein n is 2-10, such as 3-10, such as 4-10, such as preferably 5-10.
E14. An electrochemical sensor according to any one of embodiments E5-E13, wherein said optionally substituted alkyl, optionally substituted heteroalkyl, optionally substituted cycloalkyl, optionally substituted cycloheteroalkyl, optionally substituted aryl, or optionally substituted heteroaryl comprises at least one ionic substituent and a counter-ion to form a salt.
E15. An electrochemical sensor according to embodiments E14, wherein said ionic substituent is selected from the group consisting of ammonium, sulfonium, phosphonium and sulfonate.
E16. An electrochemical sensor according to any one of embodiment E14, wherein said counter-ion is selected from the group consisting of chloride, bromide, iodide, tetrafluorborate, hexafluorophosphate, tetraphenylborate, perchlorate, tetrakis(perfluoro-tert-butoxy)aluminate, thiocyanide, and trifluoroacetate, or tetrabutylammonium, tetrabutylphosphonium, potassium, sodium, magnesium, and lithium.
E17. An electrochemical sensor according to any one of embodiments E5-E16, wherein
   - R¹ is hydrogen,
   - R² is a substituted C₁-C₁₀ alkyl, comprising at least one ionic substituent and a counter-ion to form a salt.
   - R³ is an optionally substituted C₆-C₁₄ aryl or optionally substituted C₂-C₁₀ heteroaryl.
E18. An electrochemical sensor according to any of embodiments E5E17, wherein said optionally substituted alkyl comprising at least one ionic substituent and a counter-ion to form a salt, is a group of formula (III): 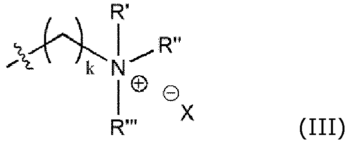 wherein k is an integer from 1-10, and
   R', R" and R"' are independently selected from optionally substituted C₁-C₅ alkyl, optionaly substituted aryl, optionally substituted heteroaryl, or any two of R', R" and R"' may be fused to form an optionally substituted cycloalkyl or heterocykloalkyl, and
   X is anionic counter-ion.
E19. An electrochemical sensor according to embodiment E18, wherein said anionic counter-ion is selected from the group consisting of chloride, bromide, iodide, tetrafluorborate, hexafluorophosphate, tetraphenylborate, perchlorate, tetrakis(perfluoro-tert-butoxy)aluminate, thiocyanide, and trifluoroacetate.
E20. An electrochemical sensor according to any of embodiments E4-E19, wherein said aryl or heteroaryl is selected from the group consisting of phenyl, naphtyl, furanyl, pyrrolyl, imidazolyl, pyrazolyl, triazolyl, pyridinyl, pyrimidinyl, pyridazinyl, pyrazinyl, thiophenyl, oxazolyl, and thiazolyl, preferably phenyl.
E21. An electrochemical sensor according to any of embodiments E5-E20, wherein said cycloalkyl or cycloheteroalkyl is selected from the group consisting of cyclopropanyl, cyclobutanyl, cyclopentanyl, cyclohexanyl, tetrahydrofuranyl, tetrahydropyranyl, tetrahydrothiophenyl, tetrahydrothiopyranyl, piperidinyl, pyrrolidinyl, morpholinyl.
E22. An electrochemical sensor according to any of embodiments E5-E21, wherein said optional substituents are selected from the group consisting of hydroxyl, amine, ammonium, or sulfonate.
E23. An electrochemical sensor according to any of the preceding embodiments, wherein said aprotic solvent is selected from the group consisting of acetonitrile, dimethylcarbonate, N,N-dimethylformamide (DMF), dimethylsulfoxide (DMSO), N-methylpyrrolidone, nitromethane, 1,1,3,3-tetramethylurea, perfluorodecaline and propylene carbonate, preferably propylene carbonate.
E24. An electrochemical sensor according to any of the preceding embodiments, arranged so that when in use a path of the analyte when moving from the associated volume (106) to the working electrode (104) traverses the aprotic solvent comprising the oxygen scavenger.
E25. An electrochemical sensor according to any of the preceding embodiments, wherein the electrolyte is comprising an aprotic solvent.
E26. An electrochemical sensor (100) according to any of the preceding embodiments, wherein a sensor response (sensitivity) and/or a baseline signal is substantially stable over extended periods of time, such as substantially stable over 30 days, such as 60 days, such as 90 days, such as wherein the response signal may be within ±25 % of an initial value for at least 90 days.
E27. An electrochemical sensor (100) according to any of the preceding embodiments, wherein the electrolyte, such as the electrolyte in the sensing chamber (120),comprises the oxygen scavenger, such as wherein the electrolyte is comprising the aprotic solvent comprising the oxygen scavenger.
E28. An electrochemical sensor according to any of the preceding embodiments, further comprising:
   - a pre-chamber (110) having a pre-chamber opening (112) towards the associated volume (106),
   wherein the sensing chamber (120) is being placed adjacent the pre-chamber (110) or partially surrounded by the pre-chamber (110),
   wherein the sensing chamber opening (122) is towards the pre-chamber (110).
   wherein the sensing chamber opening membrane (124) is arranged so as to separate the sensing chamber volume (126) from a pre-chamber volume (116), said pre-chamber volume (116) being within the pre-chamber (110).
E29. An electrochemical sensor according to embodiment E28, wherein the aprotic solvent comprising the oxygen scavenger is at least partially comprised (optionally with aprotic solvent comprising oxygen scavenger being in both pre-chamber and sensing chamber), such as fully comprised, within the pre-chamber (110).
E30. An electrochemical sensor according to embodiment E29, where the aprotic solvent comprising the oxygen scavenger is arranged for hindering oxygen in passing into the sensing chamber volume (126).

An electrochemical sensor according to any of the preceding embodiments, wherein the analyte is a gaseous analyte.
E31. An electrochemical sensor according to any of the preceding embodiments, wherein the analyte is nitrous oxide (N₂O).
E32. An electrochemical sensor according to any of the preceding embodiments, wherein the analyte is carbon dioxide (CO₂).
E33. An electrochemical sensor according to any of the preceding embodiments, wherein the analyte is any one of nitrous oxide (N₂O), carbon dioxide (CO₂), hydrogen (H₂), nitric oxide (NO), methane (CH₄), nitrogen (N₂), mono-nitrogen oxides (NOx) and/or hydrogen sulfide (H₂S).
E34. An electrochemical sensor according to any of the preceding embodiments, wherein the working electrode (104) comprises indium (In).
E35. An electrochemical sensor according to any of the preceding embodiments, wherein the working electrode (104) comprises any one of: indium (In), platinum (Pt), gold (Au), silver (Ag), palladium (Pd), iridium (Ir) and/or carbon.
E36. An electrochemical sensor according to any of the preceding embodiments, wherein the sensor comprises
   - a pre-chamber (110) having a pre-chamber opening (112) towards the associated volume (106), where a pre-chamber membrane (114) is placed in the pre-chamber opening,
   - a sensing chamber (120) being placed adjacent the pre-chamber (110) or partially surrounded by the pre-chamber (110), the sensing chamber (120) comprising,
      - A working electrode (104),
      - A reference electrode (108), and
      - An electrolyte,
      where the sensing chamber (120) has a sensing chamber opening (122) towards the pre-chamber (110), where a sensing chamber opening membrane (124) is placed in the sensing chamber opening (122),
   wherein the pre-chamber membrane (114) is permeable to the analyte and is arranged so as to separate the associated volume (106) from a pre-chamber volume (116), said pre-chamber volume being within the pre-chamber (110), and
   wherein the sensing chamber opening membrane (124) is permeable to the analyte and is arranged so as to separate the pre-chamber volume (116) from a sensing chamber volume (126), said sensing chamber volume being within the sensing chamber (120),
   where the pre-chamber (110) comprises an aprotic solvent comprising an oxygen scavenger,
   wherein the aprotic solvent comprising the oxygen scavenger is at least partially comprised, such as fully comprised, within the pre-chamber (110), such as said aprotic solvent comprising said oxygen scavenger being arranged for hindering oxygen in passing into the sensing chamber volume (126).
E37. An electrochemical sensor according to any of the preceding embodiments, wherein the sensing chamber membrane (124), is made of teflon.
E38. An electrochemical sensor according to embodiment E36, wherein the pre-chamber membrane (114), is made of teflon.
E39. An electrochemical sensor according to any of the preceding
   embodiments, wherein the aprotic solvent is propylene carbonate, wherein the oxygen scavenger is a phosphine, such as di-phenylphosphine, and
   wherein an ionic compound is dissolved in the electrolyte, the ionic compound optionally being chosen from the group comprising:
   - tetrabutylammonium iodide (TBA-I),
   - tetrabutylammonium chlorate (TBA-Cl),
   - tetrabutylammonium flouroborate (TBA-BF₄) and/or
   - tetrabutylammonium perchlorate (TBA-ClO₄).
E40. An electrochemical sensor according to any of the preceding embodiments, wherein the sensor response and/or the baseline signal is substantially stable over extended periods of time.
E41. An electrochemical sensor according to any of the preceding embodiments, wherein the electrolyte comprises propylene carbonate.
E42. An electrochemical sensor according to any of embodiments E28-E30, E36 and/or E38, wherein the pre-chamber has a volume of at least 0.5 milliliter.
E43. An electrochemical sensor according to any of the preceding embodiments, wherein an ionic compound is dissolved in the electrolyte, the ionic compound comprising tetrabutylammonium.
E44. An electrochemical sensor according to any of the preceding embodiments, wherein an ionic compound is dissolved in the electrolyte, the ionic compound being chosen from the group comprising
   - tetrabutylammonium iodide (TBA-I),
   - tetrabutylammonium chlorate (TBA-Cl),
   - tetrabutylammonium flouroborate (TBA-BF₄) and/or tetrabutylammonium perchlorate (TBA-ClO4).
E45. An electrochemical sensor according to any of the preceding embodiments, wherein the sensor is suited for measuring in any one of:
   - a gas,
   - a liquid.
E46. A sensor system comprising the sensor according to any of the preceding embodiments, and further comprising
   - a voltage generating means for holding the working electrode at a first voltage with respect to the reference electrode, such that anlayte may be reduced at the working electrode causing a current to flow through the electrode,
   - a current sensing means for sensing the presence of the current.
E47. A method (440) for electrochemically sensing an analyte in an associated volume, the method comprising
   - providing (s444) a sensing chamber, the sensing chamber comprising,
      ▪ A working electrode (104),
      ▪ A reference electrode (108), and
      ▪ An electrolyte,
      where the sensing chamber has a sensing chamber opening (122), where a sensing chamber opening membrane is placed in the sensing chamber opening,
   wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
   wherein the electrochemical sensor (100) comprises an aprotic solvent comprising an oxygen scavenger, the method further comprising reducing or oxidizing (s446) the analyte at the working electrode and measuring (s448) the corresponding current flowing through the working electrode.
E48. A method according to embodiment [previous embodiment], the method comprising
   - providing (s442) a pre-chamber having a pre-chamber opening towards the associated volume, where a pre-chamber membrane is placed the pre-chamber opening,
   - providing (s444) a sensing chamber being placed adjacent the pre-chamber or partially surrounded by the pre-chamber, the sensing chamber comprising,
      ▪ A working electrode (104),
      ▪ A reference electrode (108), and
      ▪ An electrolyte,
      where the sensing chamber has a sensing chamber opening towards the pre-chamber, where a sensing chamber opening membrane is placed in the sensing chamber opening,
   wherein the pre-chamber membrane (114) is permeable to the analyte and is arranged so as to separate the associated volume (106) from a pre-chamber volume (116), said pre-chamber volume being within the pre-chamber (110), and
   wherein the sensing chamber opening membrane (124) is permeable to the analytes and is arranged so as to separate the pre-chamber volume (116) from a sensing chamber volume (126), said sensing chamber volume being within the sensing chamber (120),
   where the pre-chamber (110) comprises an aprotic solvent comprising an oxygen scavenger, the method further comprising reducing or oxidizing (s446) the analyte at the working electrode and measuring (s448) the corresponding current flowing through the working electrode.
E49. A method according to any one of embodiments E47-E48, wherein said aprotic solvent comprising said oxygen scavenger being arranged for hindering oxygen in passing from the associated volume (106) to the working electrode (104)
E50. Use of an electrochemical sensor (100) according to any one of embodiments E1-E45 or a sensor system according to embodiment E46 for sensing the presence of an analyte in an associated volume (106).

For the above embodiments E1-E50, it may be understood that reference to preceding 'embodiments' may refer to preceding embodiments within embodiments E1-E15.

## Claims

1. An electrochemical sensor (100) for sensing an analyte in an associated volume (106), the sensor comprising
- a sensing chamber (120), the sensing chamber (120) comprising,
- A working electrode (104),
- A reference electrode (108), and
- An electrolyte,
where the sensing chamber (120) has a sensing chamber opening (122), where a sensing chamber opening membrane (124) is placed in the sensing chamber opening (122),
wherein the sensing chamber opening membrane (124) is permeable to the analyte and is arranged so as to separate a sensing chamber volume (126) from an external volume (116, 106), said sensing chamber volume being within the sensing chamber (120), said external volume being outside the sensing chamber (120),
wherein
the electrochemical sensor (100) comprises an aprotic solvent comprising an oxygen scavenger, said aprotic solvent comprising said oxygen scavenger being arranged for hindering oxygen in passing from the associated volume to the working electrode (104).

2. An electrochemical sensor according to any of the preceding claims,
wherein said oxygen scavenger is an organophosphorus compound.

3. An electrochemical sensor according to any of the preceding claims,
wherein said oxygen scavenger is a phosphine.

4. An electrochemical sensor according to claim 3, wherein said phosphine
is a compound of formula (I), or salt thereof: 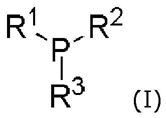 wherein R¹, R² and R³ independently are selected from the group consisting of hydrogen, optionally substituted alkyl, optionally substituted heteroalkyl, optionally substituted cycloalkyl, optionally substituted cycloheteroalkyl, optionally substituted aryl, and optionally substituted heteroaryl,
with the proviso that at least one of R¹, R² and R³ is not hydrogen.

5. An electrochemical sensor according to claim 4, wherein
- R¹ is hydrogen,
- R² is an optionally substituted alkyl, optionally substituted heteroalkyl, optionally substituted cycloalkyl or optionally substituted cycloheteroalkyl, and
- R³ is an optionally substituted aryl or optionally substituted heteroaryl.

6. An electrochemical sensor according to any one of claims 4-5, wherein said optionally substituted alkyl, optionally substituted heteroalkyl, optionally substituted cycloalkyl, optionally substituted cycloheteroalkyl, optionally substituted aryl, or optionally substituted heteroaryl comprises at least one ionic substituent and a counter-ion to form a salt.

7. An electrochemical sensor according to any one of claims 4-6, wherein
- R¹ is hydrogen,
- R² is a substituted C₁-C₁₀ alkyl, comprising at least one ionic substituent and a counter-ion to form a salt.
- R³ is an optionally substituted C₆-C₁₄ aryl or optionally substituted C₂-C₁₀ heteroaryl.

8. An electrochemical sensor according to any of claims 4-7, wherein said optionally substituted alkyl comprising at least one ionic substituent and a counter-ion to form a salt, is a group of formula (III): 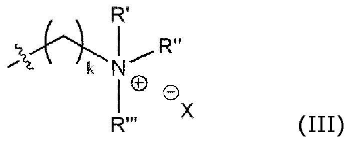 wherein k is an integer from 1-10, and
R', R" and R"' are independently selected from optionally substituted C₁-C₅ alkyl, optionaly substituted aryl, optionally substituted heteroaryl, or any two of R', R" and R"' may be fused to form an optionally substituted cycloalkyl or heterocykloalkyl, and
X is anionic counter-ion.

9. An electrochemical sensor according to any of the preceding claims,
wherein said aprotic solvent is selected from the group consisting of acetonitrile, dimethylcarbonate, N,N-dimethylformamide (DMF), dimethylsulfoxide (DMSO), N-methylpyrrolidone, nitromethane, 1,1,3,3-tetramethylurea, perfluorodecaline and propylene carbonate, preferably propylene carbonate.

10. An electrochemical sensor (100) according to any of the preceding claims, wherein the electrolyte, such as the electrolyte in the sensing chamber (120), comprises the oxygen scavenger, such as wherein the electrolyte is comprising the aprotic solvent comprising the oxygen scavenger.

11. An electrochemical sensor according to any of the preceding claims,
further comprising:
- a pre-chamber (110) having a pre-chamber opening (112) towards the associated volume (106),
wherein the sensing chamber (120) is being placed adjacent the pre-chamber (110) or partially surrounded by the pre-chamber (110),
wherein the sensing chamber opening (122) is towards the pre-chamber (110).
wherein the sensing chamber opening membrane (124) is arranged so as to separate the sensing chamber volume (126) from a pre-chamber volume (116), said pre-chamber volume (116) being within the pre-chamber (110),
wherein the aprotic solvent comprising the oxygen scavenger is at least partially comprised, such as fully comprised, within the pre-chamber (110).

12. An electrochemical sensor according to any of the preceding claims,
wherein the analyte is nitrous oxide (N₂O).

13. An electrochemical sensor according to any of the preceding claims,
wherein the analyte is carbon dioxide (CO₂).

14. An electrochemical sensor according to any of the preceding claims,
wherein the sensing chamber membrane (124) is made of teflon.

15. Use of an electrochemical sensor (100) according to any one of the preceding claims for sensing the presence of an analyte in an associated volume (106).
